# EUROPÄISCHE PATENTSCHRIFT

(11) **EP 3 461 522 B1**
(45) Veröffentlichungstag und Bekanntmachung des Hinweises auf die Patenterteilung: **30.09.2026**
(21) Anmeldenummer: 18191218.9
(22) Anmeldetag: 28.08.2018
(51) Int. Cl.: A61M 5/34

(54) **ANLAGE ZUR HERSTELLUNG EINER MEDIZINISCHEN VORRICHTUNG**
ASSEMBLY FOR THE PRODUCTION OF A MEDICAL DEVICE
INSTALLATION DE FABRICATION D'UN DISPOSITIF MÉDICAL

(30) Priorität: 28.08.2017 DE 102017119647
(43) Veröffentlichungstag der Anmeldung: 03.04.2019
(73) Patentinhaber: BBS Automation Stuttgart GmbH, 71691 Freiberg am Neckar (DE)
(72) Erfinder: Stephan, Ingo, 27211 Bassum-Nordwohlde (DE); Kretschmer, Georg, 73635 Rudersberg (DE)
(74) Vertreter: Witte, Weller & Partner Patentanwälte mbB

(56) Entgegenhaltungen:
- EP-A1- 2 546 300
- EP-A1- 3 047 869
- WO-A1-02/068027
- DE-T5- 112007 002 751
- US-A- 4 468 223
- US-A- 6 129 710
- US-A1- 2005 222 295
- HABENICHT ET AL: "Passage, Klebegrundstoffe (Kleben : Grundlagen, Technologien, Anwendungen)", 1 January 2009, KLEBEN : GRUNDLAGEN, TECHNOLOGIEN, ANWENDUNGEN, SPRINGER, DE, PAGE(S) 31 - 48, ISBN: 978-3-540-85264-3, XP009509931

## Beschreibung

Die vorliegende Beschreibung betrifft ein Verfahren zur Herstellung einer medizinischen Vorrichtung, die einen Körper und eine daran befestigte Kanüle aufweist. Die Erfindung betrifft eine Anlage zur Herstellung einer solchen medizinischen Vorrichtung gemäß Anspruch 1.

Ein Verfahren zur Herstellung einer medizinischen Vorrichtung, insbesondere einer Einmalspritze, ist aus WO 02/068027 A1 und US 6 129 710 A bekannt. Üblicherweise wird zum Aushärten des Klebemittels eine Quecksilberdampflampe als UV-Strahlungsquelle eingesetzt. Der Energiebedarf solcher Quecksilberdampflampen ist jedoch sehr hoch und auch der Wartungsaufwand führt zu hohen Kosten. Es bestand deshalb der Wunsch, diese Quecksilberdampflampen durch energieeffizientere und wartungsärmere Strahlungsquellen zu ersetzen.

Aus DE 11 2007 002751 T5 ist eine UV-Bestrahlungsvorrichtung und ein UV-Bestrahlungsverfahren bekannt. Auch die US 2005/0222295 A1 beschreibt ein UV-System zur Aushärtung.

EP 2 546 300 A1 offenbart eine wässrige Dispersion, die durch Strahlung härtbar ist. Aus HABENICHT ET AL: "Passage, Klebegrundstoffe (Kleben : Grundlagen, Technologien, Anwendungen)", 1. Januar 2009 (2009-01-01), KLEBEN : GRUNDLAGEN, TECHNOLOGIEN, ANWENDUNGEN, SPRINGER, DE, PAGE(S) 31 - 48, XP009509931, ISBN: 978-3-540-85264-3, sind ebenfalls durch UV-Strahlung härtbare Kunststoffe bekannt.

UV-Strahlungsquellen unter Nutzung der LED-Technik können einen solchen Ersatz darstellen. Sie sind deutlich energieeffizienter und wartungsärmer. Die Erfinder haben jedoch herausgefunden, dass das Klebemittel beim Einsatz von LED-Strahlen nicht vollständig aushärtet. Dies hat zur Folge, dass das später in die Spritze eingefüllte Medikament mit dem Klebemittel interagiert, was jedoch absolut zu vermeiden ist.
Weiterhin ist die Oberfläche des Klebebereichs vollständig auszuhärten, um ein mögliches Anhaften mechanischer Komponenten zu vermeiden.

Eine Aufgabe der vorliegenden Erfindung besteht deshalb darin, eine Anlage zur Herstellung einer medizinischen Vorrichtung bereitzustellen, die energieeffizient arbeitet und ohne dass später eine Wechselwirkung mit dem Medikament in der Vorrichtung auftritt und mechanische Komponenten anhaften.

Diese Aufgabe wird bei dem eingangs genannten Verfahren durch die Anlage nach Anspruch 1 dadurch gelöst, dass zum einen eine LED-Einrichtung verwendet wird, und dass zum anderen die Beaufschlagung des Klebemittels mit UV-Strahlung in einer sauerstoffreduzierten Umgebung stattfindet, wobei die UV-Strahlung von einer LED Einrichtung erzeugt wird.

Die Erfinder haben festgestellt, dass das Klebemittel während des Aushärtungs-Prozesses mit dem vorhandenen Sauerstoff reagiert und ein vollständiges Aushärten des Klebemittels verhindert. Das Reduzieren des Sauerstoffgehalts im Bereich des Klebemittels ermöglicht nun das vollständige Aushärten mit der UV-Strahlung einer LED-Einrichtung. "Sauerstoffreduzierte Umgebung" heißt in diesem Zusammenhang, dass der Sauerstoffgehalt gering, vorzugsweise jedoch null ist. Bevorzugt ist also eine sauerstofffreie Umgebung vorgesehen.

Die Aufgabe wird damit vollständig gelöst.

Bei einer bevorzugten Weiterbildung wird als Klebemittel ein Klebemittel mit dem Namen Loctite^{®} 3345 verwendet, insbesondere bei der Klebeverbindung von Glas und Metall.

Dies hat den Vorteil, dass keine neuen Zulassungs- bzw. Validierungsverfahren für den Herstellungsprozess erforderlich sind, da dieses Klebemittel bereits im Einsatz ist und die entsprechenden Zulassungen bzw. Validierungen bereits besitzt. Somit ist es möglich, den bisherigen Herstellungsprozess auf das erfindungsgemäße Herstellungsverfahren umzustellen, ohne dass neue Zulassungs- bzw. Validierungsprozesse erforderlich wären.

Gemäß der Erfindung erfolgt das Beaufschlagen des Klebemittels mit UV-Strahlung in einer Kammer. Dabei wird der Körper mit der daran befestigten Kanüle über ein Transportsystem, vorzugsweise stehend oder liegend, auf einem Werkstückträger in die Kammer eingebracht, sodass zumindest der mit Klebemittel versehene Abschnitt in der Kammer liegt. Denkbar ist jedoch auch, dass der Körper mit der Kanüle über ein Greifersystem in die Kammer eingebracht wird. Bevorzugt wird ein gasförmiges Medium in die Kammer eingebracht, welches den Sauerstoff in einem oberen oder unteren räumlichen Bereich, Aushärtungsbereich, der Kammer verdrängt, wobei das Klebemittel der medizinischen Vorrichtung in diesem Aushärtungsbereich liegt. Weiter wird die UV-Strahlung auf den Aushärtungsbereich gerichtet. Bevorzugt wird als gasförmiges Medium ein Inertgas, vorzugsweise Stickstoff, verwendet. Alternativ wird die Kammer vorgesehen, in der die Aushärtung unter Vakuum erfolgt.

Alternativ, jedoch nicht gemäß Erfindung, erfolgt das Beaufschlagen des Klebemittels mit UV-Strahlung ohne Kammer, wobei die Reduzierung des Luftsauerstoffs über das Zustellen einer mit gasförmigem Medium gefüllten Glocke erfolgt oder der Ummantelung des Klebebereichs durch ein gasförmiges Medium, welches mittels einer Düse zugeführt wird.

Diese Maßnahmen haben sich als besonders vorteilhaft herausgestellt. Der Einsatz einer Kammer, Glocke oder Ummantelung, die mit einem Inertgas, beispielsweise Stickstoff gefüllt bzw. hergestellt wird, ist prozesstechnisch einfach realisierbar.

Ein weiterer Vorteil dieser Maßnahmen kann darin gesehen werden, dass sie ermöglichen, auch mehrere medizinische Vorrichtungen gleichzeitig in einer Kammer mit UV-Strahlung einer LED-Einrichtung zu beaufschlagen.

Die vorgenannten Verfahrensschritte lassen sich im Wege einer Fließfertigung ausführen, wobei Werkstückträger für den Transport der Spritze sorgen.

Die der Erfindung zugrunde liegende Aufgabe wird gemäß Anspruch 1 von einer Fließfertigungs-Anlage zur Herstellung medizinischer Vorrichtungen gelöst, die zusätzlich eine erste Station zum Einbringen der Kanüle in eine Öffnung im Körper, eine zweite Station zum Applizieren eines Klebemittel, eine dritte Station, die die Kammer, die LED-Einrichtung und die Gaszuführ-Einrichtung aufweist, und eine Transporteinrichtung, die ausgebildet ist, die medizinische Vorrichtung von der ersten Station, zur zweiten Station und dann zur dritten Station zu transportieren, wobei sich die medizinische Vorrichtung zum Transport auf einem Werkstückträger befindet.

Eine solche Anlage ist durch Einsatz von LED-Technologie sehr energieeffizient und wartungsarm, ohne die Qualität der hergestellten medizinischen Vorrichtung zu beeinträchtigen.

Gemäß der Erfindung ist die Kammer derart ausgebildet, dass die medizinische Vorrichtung, vorzugsweise stehend oder liegend, zumindest teilweise in der Kammer aufgenommen wird, und die LED-Einrichtung so vorgesehen ist, vorzugsweise außerhalb an der Kammer derart angebracht ist, dass die von der LED-Einrichtung ausgestrahlte UV-Strahlung das Klebemittel erreicht. Bevorzugt weist die LED-Einrichtung ein oder mehrere LEDs auf, und weist die Gaszuführ-Einrichtung zumindest einen Gasspeicher, ein steuerbares Ventil und eine Gasleitung auf.

Weiter bevorzugt ist an der Kammer ein Sensor zur Erfassung des Sauerstoffgehalts vorgesehen.

Dies hat den Vorteil, dass der Sauerstoffgehalt überprüft und entsprechend bei Bedarf Inertgas zugeführt werden kann.

Die vorliegende Beschreibung umfasst auch eine Vorrichtung mit einem Körper aus Glas oder Kunststoff und einer daran mit einem UV-aushärtbaren Klebemittel angebrachten Kanüle aus Metall oder Kunststoff, wobei das Klebemittel in einer sauerstoffreduzierten Umgebung mit UV-Licht eines LED-Systems ausgehärtet wurde.

An dieser Stelle sei noch angemerkt, dass im Rahmen der vorliegenden Beschreibung die Begriffe Kanüle, Hohlnadel oder Nadel gleichbedeutende Begriffe sind.

Weitere Vorteile und Ausgestaltungen der Erfindung ergeben sich aus der Beschreibung und der beiliegenden Zeichnung. Dabei zeigen:
Figur 1 eine schematische Darstellung einer medizinischen Vorrichtung in Form einer Spritze;
Figur 2 eine Detailansicht der in Figur 1 gezeigten Spritze;
Figuren 3A-C verschiedene Ausgestaltungen einer Kammer oder Glocke, in der der Aushärtungs-Prozess erfolgt;
Figur 4 eine schematische Darstellung einer Anlage gemäß der Erfindung zur Herstellung einer medizinischen Vorrichtung; und
Figur 5 ein schematisches Diagramm zur Erläuterung des hierin beschriebenen Verfahrens.

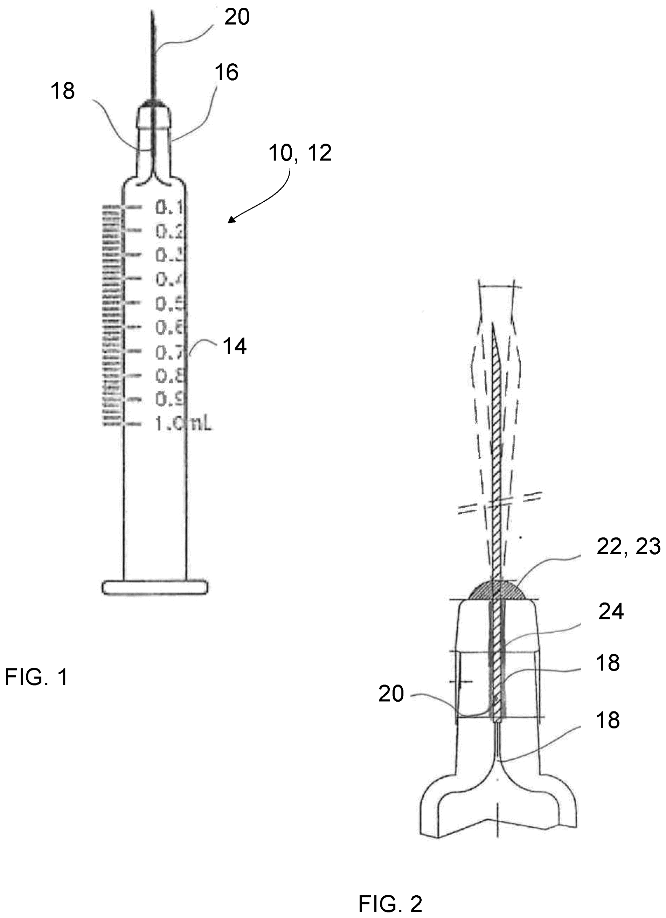

In Figur 1 ist eine medizinische Vorrichtung schematisch dargestellt und mit dem Bezugszeichen 10 gekennzeichnet. Bevorzugt handelt es sich bei der medizinischen Vorrichtung 10 um eine Spritze, insbesondere eine Einmalspritze 12.

Eine solche Spritze 12 weist einen Körper mit einem zylindrischen Hohlraum 14 zur Aufnahme eines Medikaments und einen Düsen- bzw. Konusbereich 16 auf. Üblicherweise ist die Spritze 12 aus Glas hergestellt, wobei auch Kunststoff als Material verwendet werden könnte.

Der Konusbereich 16 weist einen in Längsrichtung der Spritze verlaufenden Kanal 18 auf, in den eine Kanüle bzw. Hohlnadel 20 eingebracht ist. Die aus Metall oder Kunststoff gefertigte Kanüle 20 ist mit dem Konusbereich 16 über ein-Klebemittel 22 fest verbunden. Als Klebemittel kommt bevorzugt das Produkt Loctite^{®} 3345 zum Einsatz. Es handelt sich hierbei um einen lichthärtenden Sofortklebstoff auf Acrylatbasis, der zum Kleben von Glas und Metallen gut geeignet ist.

Aus Vereinfachungsgründen wurde in Figur 1 der Kolben zum Applizieren des in dem Hohlraum vorhandenen Medikaments durch die Kanüle weggelassen.

In Figur 2 ist der Konusbereich 16 der Spritze 12 vergrößert dargestellt. In der Darstellung ist das Klebemittel 22, in Form des Klebstoffs Loctite^{®} 3345, gekennzeichnet durch das Bezugszeichen 23, gut zu erkennen. Das Klebemittel 22 bildet einen kugelsegmentartigen Bereich auf dem Ende des Konusbereichs 16 und erstreckt sich zumindest teilweise in den Kanal 18, wobei dieser Bereich innerhalb des Kanals 18 mit dem Bezugszeichen 24 gekennzeichnet ist. In dem vorliegenden Beispiel reicht das Klebemittel 22 bis zum unteren Ende der Hohlnadel 20, also fast vollständig durch den Kanal 18.

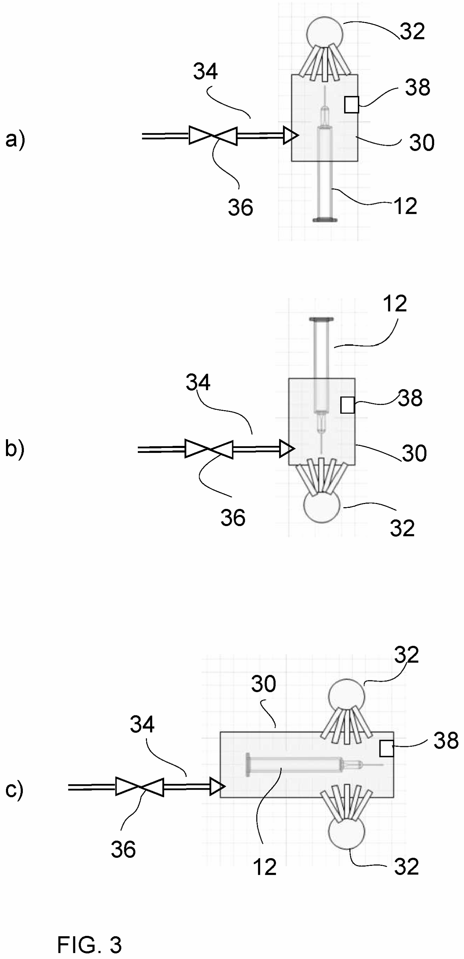

Wie bereits erläutert wird das Klebemittel 22 durch UV-Licht ausgehärtet. Mit Bezug auf die Figuren 3A bis C werden nachfolgend drei verschiedene Möglichkeiten dargestellt, wie das Aushärten erfolgen kann. An dieser Stelle sei jedoch angemerkt, dass andere Möglichkeiten denkbar sind.

In Figur 3A ist eine Kammer 30 schematisch dargestellt, die einen zumindest teilweise abgeschlossenen Innenraum definiert, in den eine Spritze 12 eingebracht werden kann. Im vorliegenden Fall wird die Spritze 12 stehend in die Kammer 30 eingebracht, sodass zumindest der Konusbereich 16 und die Hohlnadel 20 innerhalb der Kammer liegen.

Zum Bestrahlen des Klebemittels wird eine LED-Einrichtung 32 verwendet, die ein oder mehrere LEDs aufweist. Die LED-Einrichtung 32 ist oberhalb der Kammer 30 angeordnet, sodass die Strahlung der LED von oben durch eine geeignete Öffnung auf das Klebemittel trifft.

Die Kammer 30 ist mit einer Gaszuführ-Einrichtung 34 verbunden, die ein Ventil 36 aufweist. Über diese Gaszuführ-Einrichtung 34 lässt sich gesteuert über das Ventil 36 ein Inertgas in den Innenraum der Kammer 30 einbringen. Bevorzugt wird als Inertgas Stickstoff verwendet. Der einströmende Stickstoff füllt die Kammer und verdrängt damit in der Kammer vorhandenen Sauerstoff. Wichtig ist, dass der Bereich mit dem Klebemittel 22 im sauerstoffreduzierten, d. h. Stickstoff gefüllten Bereich der Kammer liegt.

Zur Überprüfung, ob der Sauerstoff ausreichend aus der Kammer 30 verdrängt wurde, weist die Kammer 30 einen Sensor 38 auf, der ausgelegt ist, den Sauerstoffgehalt vorzugsweise in Höhe des Konusbereichs 16 der Spritze 12 zu messen. Abhängig von dem gemessenen Sauerstoffgehalt wird über das Ventil 36 gesteuert Stickstoff zugeführt.

In dieser sauerstoffreduzierten Umgebung innerhalb der Kammer 30 wird das Klebemittel 22 über die UV-Strahlung der LED-Einrichtung 32 ausgehärtet, ohne dass während dieses Prozesses das Klebemittel 22 durch Sauerstoff beeinflusst wird. Damit kann das Klebemittel 22 während einer vordefinierten Zeitdauer, vollständig Aushärten.

Nach der vordefinierten Zeitdauer kann die Spritze 12 dann die Kammer 30 wieder verlassen, um einem nächsten Prozessschritt zugeführt zu werden.

In Figur 3B ist eine alternative Ausgestaltung der Kammer 30 dargestellt, wobei der Unterschied darin besteht, dass die Spritze 12 mit ihrem Konusbereich 16 nach unten gerichtet in die Kammer eingebracht wird. Um das Klebemittel bestrahlen zu können, ist die LED-Einrichtung 32 an der Unterseite der Kammer vorzugsweise außerhalb vorgesehen. Die Funktionsweise dieser alternativen Ausgestaltung ist jedoch gleich zu jener, die mit Bezug auf die Figur 3A beschrieben wurde.

In Figur 3C ist eine weitere alternative Ausgestaltung der Kammer 30 dargestellt. Hier wird die Spritze 12 liegend in die Kammer eingebracht, wobei dies erfordert, dass mehrere LED-Einrichtungen 32 vorgesehen sind, um den Konusbereich 16 mit dem Klebemittel 22 optimal bestrahlen zu können. Bei der vorliegenden Ausführungsform sind zwei LED-Einrichtungen 32 einander gegenüberliegend vorgesehen. Die Funktionsweise selbst verändert sich dadurch jedoch nicht.

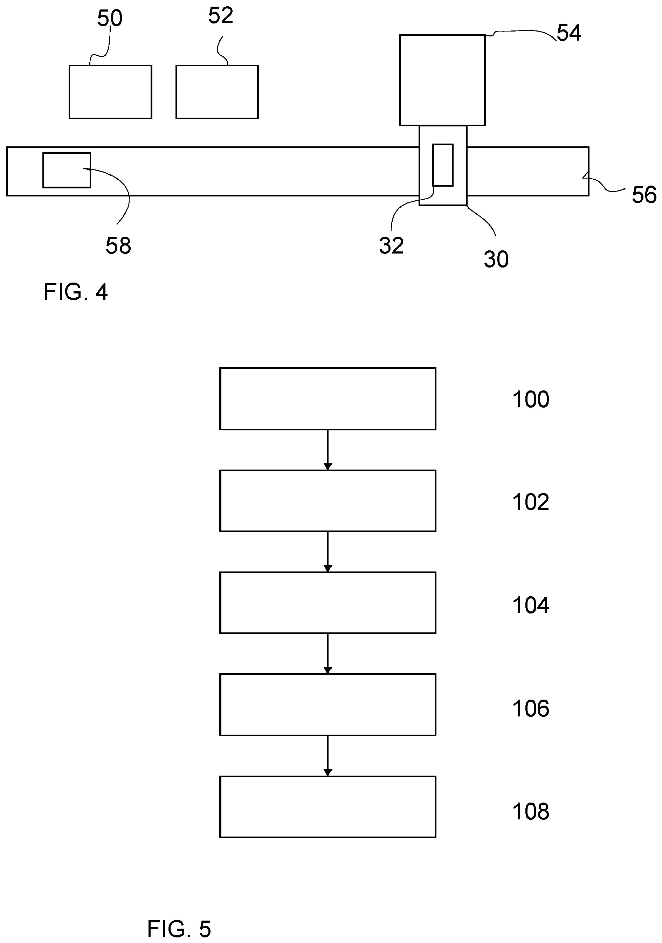

In Figur 4 ist eine Anlage zur Herstellung einer Spritze 12 schematisch dargestellt. Die Anlage umfasst eine erste Station 50, eine zweite Station 52 und eine dritte Station 54, die entlang einer Transporteinrichtung 56, die Werkstückträger 58 transportiert, angeordnet sind.

Die erste Station 50 ist ausgelegt, die Hohlnadel bzw. Kanüle 20 in den Kanal 18 der Spritze 12 einzubringen. Die Einheit aus Spritze und Kanüle befindet sich auf dem Werkstückträger und wird zu der sich anschließenden zweiten Station transportiert.

Die zweite Station 52 ist ausgelegt, dass Klebemittel 22 zu applizieren. Der Werkstückträger 58 transportiert dann Spritze und Kanüle zu der sich anschließenden dritten Station 54.

Die dritte Station 54 weist die Kammer 30 mit der LED-Einrichtung 32 und der Gaszuführ-Einrichtung 34 auf, um dort den zuvor beschriebenen Aushärtungs-Prozess durchzuführen. Nach dem Aushärtungs-Prozess wird die Spritze mit der nun fest eingeklebten Kanüle über den Werkstückträger 58 aus der Kammer transportiert und einer sich anschließenden Weiterverarbeitung zugeführt.

An dieser Stelle sei angemerkt, dass ein oder mehrere Stationen 50-54 zu einer Station zusammengeführt werden können.

In Figur 5 ist ein Blockdiagramm zur Erläuterung des zuvor beschriebenen Verfahrens dargestellt. In einem ersten Schritt 100 wird der Körper mit dem zylindrischen Hohlraum 14 der Spritze 12 bereitgestellt. Im nächsten Schritt 102 wird die Kanüle bzw. Hohlnadel 20 bereitgestellt. Im nächsten Schritt 104 wird die Hohlnadel 20 in den Kanal 18 eingebracht. Im nächsten Schritt 106 wird das Klebemittel 22 appliziert und im Schritt 108 wird das Klebemittel 22 in einer sauerstoffreduzierten Umgebung mittels einer LED-Einrichtung bestrahlt und dadurch ausgehärtet. An dieser Stelle sei jedoch angemerkt, dass der Prozess des Aushärtens teilweise auch außerhalb der sauerstoffreduzierten Umgebung erfolgen kann, bspw. können zusätzliche Schritte Vorhärten bzw. Nachhärten außerhalb der sauerstoffreduzierten Umgebung vorgesehen werden. Das beschriebene Verfahren wird im Zuge einer Fließfertigung ausgeführt, das heißt, die Bauteile wie Spritze und Kanüle werden durch ein oder mehrere Stationen mittels Werkstückträgern transportiert, so dass die einzelnen Schritte aufeinanderfolgend ausgeführt werden können.

Das Ergebnis dieses Verfahrens ist eine Spritze die trotz Bestrahlung durch eine LED-Einrichtung ein vollständig ausgehärtetes und ausreagiertes Klebemittel aufweist, sodass eine Wechselwirkung zwischen Klebemittel und Medikament sowie ein Anhaften mechanischer Komponenten nicht mehr stattfinden kann.

Entscheidend bei diesem Verfahren ist, dass die Bestrahlung in einer sauerstoffreduzierten Umgebung erfolgt, sodass während des Aushärtungs-Prozesses das Klebemittel nicht mit Sauerstoff reagieren kann. Diese sauerstoffreduzierte Umgebung lässt sich sehr einfach innerhalb einer Kammer erreichen, die mit einem Inertgas, vorzugsweise Stickstoff geflutet wird.

Selbstverständlich sind auch andere Möglichkeiten zur Bereitstellung einer sauerstoffreduzierten Umgebung denkbar. Beispielsweise könnte das Aushärten auch in einem Vakuum erfolgen. Denkbar wäre auch, das Klebemittels mit UV-Strahlung ohne Nutzung einer Kammer zu beaufschlagen, wobei die Reduzierung des Luftsauerstoffs über das Zustellen einer mit gasförmigem Medium gefüllten Glocke erfolgt oder der Ummantelung des Klebebereichs durch ein gasförmiges Medium, welches mittels einer Düse zugeführt wird.

Insgesamt zeigt sich, dass der Ersatz von Quecksilberdampflampen durch moderne LEDs möglich ist, ohne das bisher eingesetzte Klebemittel ersetzen zu müssen. Trotz des etwas größeren Aufwandes durch die Schaffung einer sauerstoffreduzierten Umgebung beim Aushärtungs-Prozess überwiegen die Kostenvorteile sowohl bei der Beschaffung einer Anlage als auch während deren Betriebs.

## Patentansprüche

1. Anlage zur Herstellung medizinischer Vorrichtungen, nämlich von Spritzen (12), insbesondere Einwegspritzen (12), die jeweils einen Körper aus Glas mit einem zylindrischen Hohlraum zur Aufnahme eines Medikaments und eine Kanüle aus Metall oder Kunststoff aufweisen, mit
einer ersten Station (50) zum Einbringen der Kanüle in eine Öffnung im Körper, einer zweiten Station (52) zum Applizieren eines unter UV-Strahlung aushärtbaren Klebemittels in den Bereich der Öffnung, sodass sich das Klebemittel zumindest teilweise in einen sich in Längsrichtung der Spritze erstreckenden Kanal (18) erstreckt, der in einem Konusbereich (16) des Körpers vorliegt und dass das Klebemittel einen kugelsegmentartigen Bereich auf dem Ende des Konusbereichs (16) bildet,
einer dritten Station (54), die eine Kammer (30), eine LED-Einrichtung (32), die UV-Strahlung zum Aushärten des Klebemittels erzeugt, und eine Gaszuführ-Einrichtung (34) oder eine Einrichtung zur Erzeugung eines Vakuums in der Kammer aufweist, und die dazu ausgebildet ist, das Klebemittel auszuhärten, um die Kanüle fest mit dem Konusbereich (16) zu verbinden, wobei die Gaszuführ-Einrichtung (34) ausgebildet ist, ein gasförmiges Medium in die Kammer einzubringen, welches den Sauerstoff in einem oberen oder unteren räumlichen Aushärtungsbereich der Kammer verdrängt, und wobei in diesem Aushärtungsbereich das Klebemittel der medizinischen Vorrichtung liegt, und
einer Transporteinrichtung (56), die ausgebildet ist, die medizinische Vorrichtung von der ersten Station über die zweite Station zur dritten Station zu transportieren, wobei sich die medizinische Vorrichtung während des Transports auf einem Werkstückträger (58) befindet;
wobei die Kammer (30) derart ausgebildet ist, die medizinische Vorrichtung stehend mit nach oben weisendem Konusbereich (16) zumindest teilweise aufzunehmen und die LED-Einrichtung (32) so angeordnet ist, dass die UV-Strahlung der LED auf den Aushärtungsbereich gerichtet ist, und
wobei die Anlage eine Fließfertigungsanlage ist.

2. Anlage nach Anspruch 1, **dadurch gekennzeichnet, dass** die LED-Einrichtung (32) ein oder mehrere LEDs aufweist und dass die Gaszuführ-Einrichtung (34) zumindest einen Gas-Speicher, ein steuerbares Ventil (36) und eine Gasleitung aufweist.

3. Anlage nach einem der Ansprüche 1 bis 2, **dadurch gekennzeichnet, dass** an der Kammer (30) ein Sensor (38) zur Erfassung des Sauerstoffgehalts vorgesehen ist, wobei die Gaszuführungs-Einrichtung abhängig vom erfassten Sauerstoffgehalt Gas zuführt.

## Claims

1. System for manufacturing medical devices, namely syringes (12), in particular disposable syringes (12), each comprising a body made of glass with a cylindrical cavity for receiving a medicament and a cannula made of metal or plastic, comprising
- a first station (50) for inserting the cannula into an opening in the body,
- a second station (52) for applying an adhesive curable by UV radiation into the region of the opening, such that the adhesive extends at least partially into a channel (18) extending in the longitudinal direction of the syringe, the channel being provided in a cone region (16) of the body, and such that the adhesive forms a spherical-segment-shaped region on the end of the cone region (16),
- a third station (54), comprising a chamber (30), an LED device (32) generating UV radiation for curing the adhesive, and a gas supply device (34) or a device for generating a vacuum in the chamber, and configured to cure the adhesive in order to firmly connect the cannula to the cone region (16), wherein the gas supply device (34) is configured to introduce a gaseous medium into the chamber, which displaces the oxygen in an upper or lower spatial curing region of the chamber, and wherein the adhesive of the medical device is located in this curing region, and
- a transport device (56) configured to transport the medical device from the first station via the second station to the third station, wherein the medical device is located on a workpiece carrier (58) during transport;
wherein the chamber (30) is configured to at least partially receive the medical device in an upright position with the cone region (16) pointing upward, and the LED device (32) is arranged such that the UV radiation of the LED is directed toward the curing region, and wherein the manufacturing system is a flow manufacturing system.

2. System according to claim 1, **characterized in that** the LED device (32) comprises one or more LEDs and that the gas supply device (34) comprises at least one gas reservoir, a controllable valve (36), and a gas line.

3. System according to one of claims 1 to 2, **characterized in that** a sensor (38) for detecting the oxygen content is provided on the chamber (30), wherein the gas supply device supplies gas depending on the detected oxygen content.

## Revendications

1. Installation destinée à la fabrication de dispositifs médicaux, à savoir de seringues (12), en particulier de seringues à usage unique (12), lesquelles présentent respectivement un corps en verre comportant une cavité cylindrique destinée à recevoir un médicament et une canule en métal ou en matière plastique, comportant
un premier poste (50) pour l'insertion de la canule dans une ouverture dans le corps, un deuxième poste (52) pour l'application d'un adhésif durcissable sous rayonnement UV dans la zone de l'ouverture, de sorte que l'adhésif s'étend au moins partiellement dans un canal (18) s'étendant dans la direction longitudinale de la seringue, lequel canal se trouve dans une zone conique (16) du corps, et que l'adhésif forme une zone en forme de segment sphérique à l'extrémité de la zone conique (16),
un troisième poste (54) qui présente une chambre (30), un appareil à DEL (32) générant un rayonnement UV pour le durcissement de l'adhésif, et un appareil d'acheminement de gaz (34) ou un appareil pour la génération d'un vide dans la chambre, et qui est conçu pour durcir l'adhésif afin de relier solidement la canule à la zone conique (16), dans laquelle l'appareil d'acheminement de gaz (34) est conçu pour introduire dans la chambre un milieu gazeux qui refoule l'oxygène dans une zone de durcissement, supérieure ou inférieure spatialement, de la chambre, et dans laquelle l'adhésif du dispositif médical se trouve dans ladite zone de durcissement, et
un appareil de transport (56) qui est conçu pour transporter le dispositif médical du premier poste au troisième poste par l'intermédiaire du deuxième poste, dans laquelle le dispositif médical se trouve sur un porte-outil (58) pendant le transport ;
dans laquelle la chambre (30) est conçue pour recevoir au moins partiellement le dispositif médical en position verticale avec la zone conique (16) orientée vers le haut, et l'appareil à DEL (32) est disposé de sorte que le rayonnement UV de la DEL est dirigé vers la zone de durcissement, et
dans laquelle l'installation est une installation de production à la chaîne.

2. Installation selon la revendication 1, **caractérisée en ce que** l'appareil à DEL (32) présente une ou plusieurs DEL **et en ce que** l'appareil d'acheminement de gaz (34) présente au moins un moyen de stockage de gaz, une vanne commandable (36) et une conduite de gaz.

3. Installation selon l'une des revendications 1 à 2, **caractérisée en ce qu'**un capteur (38) pour la détection de la teneur en oxygène est prévu au niveau de la chambre (30), dans laquelle l'appareil d'acheminement de gaz achemine du gaz en fonction de la teneur en oxygène détectée.
